# EUROPEAN PATENT SPECIFICATION

(11) **EP 1 472 246 B1**
(45) Date of publication and mention of the grant of the patent: **04.04.2007**
(21) Application number: 03737409.7
(22) Date of filing: 31.01.2003
(51) Int. Cl.: C07D 413/04, A61K 31/4245, A61P 35/00, C07D 209/60, C07D 209/70

(54) **OXADIAZOLES HAVING ANTIPROLIFERATIVE ACTIVITY**
OXADIAZOLE MIT ANTIPROLIFERATIVER WIRKUNG
OXADIAZOLES POSSEDANT UNE ACTIVITE ANTIPROLIFERATIVE

(30) Priority: 08.02.2002 IT MI20020236
(43) Date of publication of application: 03.11.2004
(73) Proprietor: Universita' Degli Studi Di Milano, 20122 Milano (IT); UNIVERSITA' DEGLI STUDI DI SASSARI, 07100 Sassari (IT)
(72) Inventor: CIGNARELLA, Giorgio, I-20131 Milano (IT); PINNA, Gerard Aime, I-07100 Sassari (IT); MURINEDDU, Gabriele, I-07100 Sassari (IT)
(74) Representative: Minoja, Fabrizio
(86) International application number: PCT/IB2003/000322
(87) International publication number: WO 2003/066628

(56) References cited:
- DATABASE CROSSFIRE BEILSTEIN [Online] Beilstein Institut zur Förderung der Chemischen Wissenschaften, Frankfurt am Main, DE; XP002242317 & KHIM. GETEROTSIKL. SOEDIN., vol. 7, 1979, page 909
- PINNA G A ET AL: "Synthesis and cytotoxicity of bis(benzo[g]indole-3- carboxamides) and related compounds" ARCHIV DER PHARMAZIE, vol. 334, no. 11, December 2001 (2001-12), pages 337-344, XP002242316

## Description

### Background of the invention

Arch. Pharm., Pharm. Med. Chem. 2001, 334, pages 337-344 discloses bis(benzo[g]indoles) bridged by CX-(CH₂)ₙN(Me)(CH₂)ₙ-CX, wherein X = O, S, H₂ and n = 2,3 and related compounds as intercalating agents endowed with antitumor activity.

### Detailed disclosure of the invention

The present invention relates to oxadiazole derivatives of general formula (**I**) 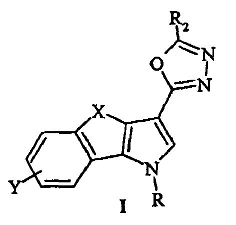 in which:
X is a CH₂, CH₂CH₂ or CH=CH group;
R is hydrogen, straight or branched C₁-C₄ alkyl, or phenyl-C₁-C₂-alkyl;
R₂ is hydrogen, straight or branched C₁-C₄ alkyl, or phenyl, optionally substituted with one more groups, which can be the same or different, selected from halogen (fluorine, chlorine, bromine, iodine), straight or branched C₁-C₄ alkyl, C₁-C₃ alkoxy, trifluoromethyl;
Y is hydrogen, halogen (fluorine, chlorine, bromine, iodine), straight or branched C₁-C₄ alkyl, C₁-C₃ alkoxy, trifluoromethyl.

C₁-C₄ Alkyl is preferably methyl or ethyl.

Phenyl-C₁-C₂-alkyl is preferably benzyl, optionally substituted on the phenyl ring with one more groups, which can be the same or different, selected from halogen (fluorine, chlorine, bromine, iodine), straight or branched C₁-C₃ alkyl, C₁-C₃ alkoxy, trifluoromethyl.

C₁-C₃ Alkoxy is preferably methoxy or ethoxy.

The invention also relates to the non toxic salts and solvates of compounds (**I**).

In compounds of formula (**I**), X is preferably a CH₂ or a CH=CH group, R is is hydrogen or methyl and R₂ is hydrogen, methyl or optionally substituted substituted phenyl, as defined above.

More preferably, R₂ is phenyl or substituted phenyl, as defined above.

Examples of preferred compounds according to the invention comprise:
3-[[1,3,4]oxadiazol-2'-yl]-1,4-dihydro-indeno[1,2-b]pyrrole;
3-[[1,3,4]oxadiazole-5'-methyl-2'-yl]-1,4-dihydro-indeno[1,2-b]pyrrole;
3-[[1,3,4] oxadiazole-5'-phenyl-2'-yl]-1,4-dihydro-indeno[1,2-b]pyrrole;
1-methyl-3-[[1,3,4] oxadiazol-2'-yl]-1,4-dihydro-indeno[1,2-b]pyrrole;
3-[[1,3,4]oxadiazol-2'-yl]-4,5-dihydro-1H-benzo[g]indole;
3-[[1,3,4]oxadiazole-5'-methyl-2'-yl]-4,5-dihydro-1H-benzo[g]indole;
3-[[1,3,4]oxadiazole-5'-phenyl-2'-yl]-4,5-dihydro-1H-benzo[g]indole;
1-methyl-3-[[1,3,4]oxadiazol-2'-yl]-1H-benzo[g]indole;
3-[[1,3,4]oxadiazol-2'-yl]-1H-benzo[g]indole;
3-[[1,3,4]oxadiazole-5'-methyl-2'-yl]-1H-benzo[g]indole;
3-[[1,3,4]oxadiazole-5'-phenyl-2'-yl]-1H-benzo[g]indole.

Compounds of formula (**I**) have cytotoxic activity and can be used as antitumor and antiproliferative medicaments.

Compounds of formula (**I**) can be prepared by reacting hydrazides of formula (**II**) (scheme 1) wherein X, Y and R are as defined above, with an orthoformate of formula **(III)**,

R₂C(OEt)₃ **(III)**

in which R₂ has the meaning defined above.

The reaction is carried out in a dipolar aprotic solvent, preferably dimethylformamide.

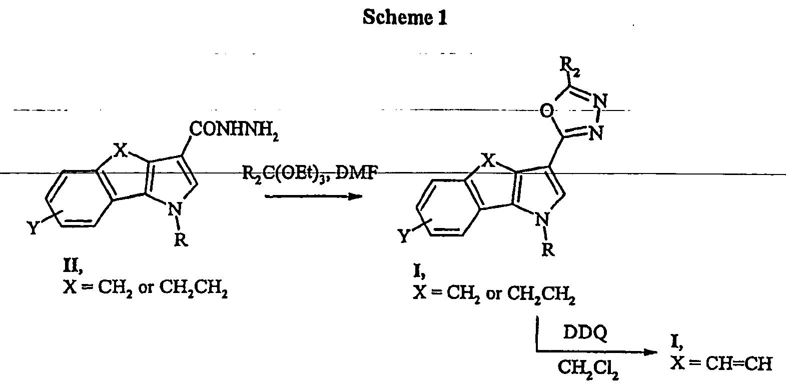

The compound of formula (II) wherein H is -CH=CH- and Y and R are hydrogen is disclosed in Chem. Heterocycl. Compd.; 15, 1979, 741.

Compounds (**I**) in which X is CH=CH can be obtained by treatment of a compound of formula (**I**), in which X is CH₂CH₂, with DDQ in CH₂CI₂.

Compounds of formula (**II**), which are novel and are a further object of the invention, can be obtained as shown in Scheme 2 (step *i*), starting from a compound of formula (**IV**), in which X is CH₂ or CH₂CH₂ and Y is as defined above, by reaction with a cyanoacetic acid ester of formula **V**

CNCH₂COOR₁ (**V**)

in which R₁ has the same meanings as R defined above except for hydrogen, to give the compounds of formula **VI**, which are then treated with hydrochloric acid gas in ether solution (step *ii),* to yield the compounds of formula **(VII)** in which R is hydrogen. The reaction which affords compounds **(VI)** is also accompanied by formation of compounds **(VIII),** which are removed with conventional techniques. Step *i*) is preferably carried out in acetone in the presence of potassium bicarbonate, but also other solvents and bases may be used. Compounds (**VII**) can be optionally alkylated at the pyrrole nitrogen (step *iii)* with an alkylsulfonate of formula (**IX**).

R₂SO₄ (**IX**)

in which R has the meanings defined above except for hydrogen. 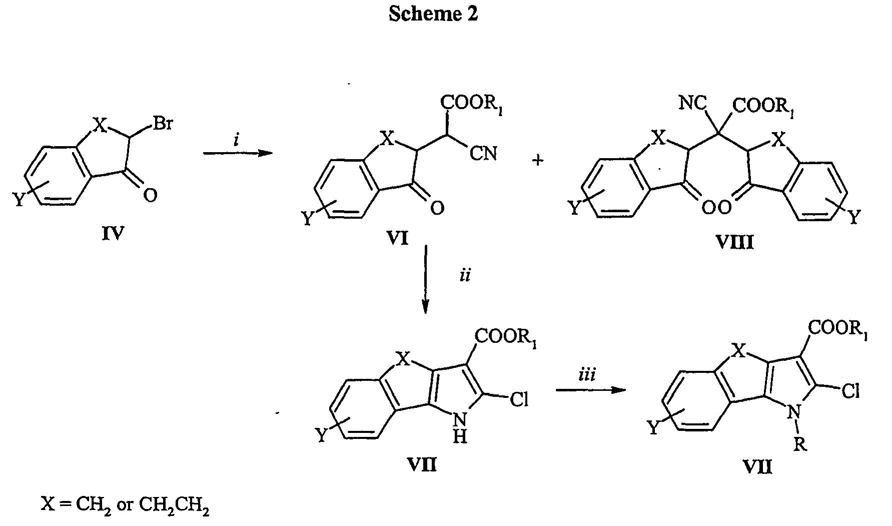
*i*) K₂CO₃, CNCH₂COOR₁, acetone
*ii*) Et₂O, gas HCI
*iii*) EtOH, KOH, acetone, R₂SO₄

Reductive dehalogenation of compounds (**VII**) with ammonium formate in methanol and Pd-C yields compounds (**X**), as shown in Scheme 3. The latter are treated with DDQ in CH₂CL₂ to obtain compounds (**X**) in which **X** is CH=CH.

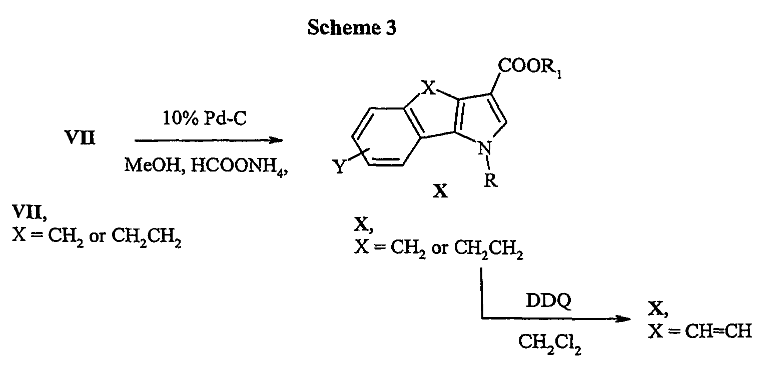

Compounds (**X**) are then reacted with hydrazine (scheme 4) to give compounds of formula (**II**).

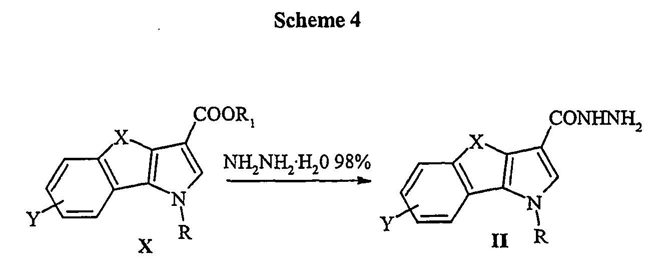

The cytotoxic activity of compounds (**I**) was evidenced in primary screening studies on 60 human tumor cell lines. Compounds (**I**) showed marked antiproliferative activity, in particular against leukemias, colon and breast tumors.

Compounds of formula (**I**) may therefore be used as antitumor medicaments, more particularly for the treatment of leukemias, colon and breast tumors. For this purpose, compounds of formula (**I**) will be formulated in admixture with suitable excipients and/or carriers according to conventional techniques. The pharmaceutical compositions of the invention may be administered through the oral, parenteral, rectal or topical route.

The therapeutical dosages will depend on a number of factors, such as the severity of the disease to treat, the weight, sex and age of the patient as well as the pharmaco-toxicological and pharmacokynetics characteristics of the selected compound of formula (**I**). In principle, the daily dosage will range from 0.01 to 10 mg /kg body weight of the patient.

The invention is illustrated in greater detail by the following examples.

### EXAMPLES

### Materials and methods

Compound (**IVa**) (α**-**bromoindan-1-one) is commercially available (Aldrich Chemical Co.), whereas compound (**IVb**) (α-bromo-tetral-1-one) is synthesized according literature [Wilds A.L., Johnson J.A.Jr., *J*. *Am. Chem. Soc.,* 68, 86-89 (1946)].

Flash chromatography was performed on silica gel Merck 60 (230-400 mesh ASTM). Thin layer chromatography (TLC) was carried out on plates (0.2 mm) Polygram^{®}SIL N-HR-/HV₂₅₄.

Melting points were determined with a Thomas-Hoover melting point capillary apparatus and are not corrected.

IR spectra were recorded on NaCl pellets (on thin films of the products in Nujol) with a Perkin Elmer 781 IR spectrophotometer and are expressed in ν cm⁻¹.

NMR spectra were recorded with an XL-200 Varian instrumentation at 200 MHz and chemical shifts are expressed in ppm.

### Example 1

### Ethyl 2-cyano-2(1-oxy-2,3-dihydro-1H-inden-2-yl)acetate (VIa)

A solution of α-bromoindan-1-one **(IVa)** (5.02 mmol) in acetone (10.9 ml) is added, drop by drop, with a suspension of CNCH₂COOEt (40 mmol) and K₂CO₃ (10 mmol) at 40-45°C. The reaction mixture is stirred at 40-45°C for lh and then cooled to room temperature. Ethyl acetate (10 ml) and water (10 ml) are added under stirring; the organic layer is separated, washed with a 10% KH₂PO₄ solution (7.5 ml) and ice (5 ml), dried over Na₂SO₄ and concentrated under reduced pressure to give a crude oil, which is subsequently distilled (150°C/1mmHg) and purified by flash chromatography (eluent: petroleum ether/ethyl acetate 8:2)

Compound (**VIb**) is prepared following the same procedure.

### Example 2

### Ethyl 1,4-dihydro-indeno[1,2-b]pyrrole-2-chloro-3-canboxylate (VIIa)

A solution of compound (**VIa**) (6.0 mmol) in ethyl ether (15 ml), cooled in ice at 0-5°C, is added with hydrochloric acid gas (1.86 g, 5.1 mmol). The solution is kept under stirring at room temperature for 24 h, then ethyl ether and hydrochloric acid excess are removed with nitrogen. The solid residue is triturated in methanol to give cream crystals.

Compound (**VIIb**) is prepared following the same procedure, but preparing the starting solution with 30 ml of ethyl ether.

### Example 3

### Ethyl 1-methyl-1,4-dihydro-indeno[1,2-b]pyrrole-2-chloro-3-carboxylate (VIIc)

0.28 g (4.99 mmol) of KOH are dissolved in a solution of compound (**VIIa**) (4.14 mmol) in EtOH (23 ml); the solvent is evaporated off under reduced pressure and the residue is dissolved in acetone (18.6 ml),then added with 0.78 ml (8.28 mmol) of Me₂SO₄. The mixture is kept under stirring at room temperature until completion of the reaction (about 30 min, TLC petroleum ether/ethyl acetate 8/2). The precipitated solid is filtered off and the solution is concentrated under reduced pressure to give an oil which solidifies upon standing.

Compound **(VIIb)** is prepared following the same procedure, starting from compound **(VIId).**

### Example 4

### Ethyl 1,4-dihydro-indeno[1,2-b]pyrrole-3-carboxylate (Xa)

A solution of 2.72 mmol of chloroester **(VIIa)** and 13.6 mmol (0.86 g) of ammonium formate in 22.44 ml of methanol is added with 0.18 g of 10% Pd-C. The mixture is kept under nitrogen stream and under stirring until completion of the reaction (about 4 hours, TLC: petroleum ether/ethyl acetate 8/2), after that is filtered and the solution is evaporated under reduced pressure.

The resulting residue is taken up into water; the resulting suspension is filtered and the precipitate is washed with ethanol.

Compound (**Xb**) is prepared following the same procedure. Compounds **(Xc)** and **(Xd)** are purified by extraction of the evaporation residue aqueous solution with ether, evaporation of the ether phase and distillation under low pressure.

### Example 5

### Ethyl 1-methyl-1H-benzo[g]indole-3-carboxylate (X_{f})

Compound **(Xd)** (0.67 g, 2.66 mmol) is dissolved in 10 ml of CH₂Cl₂, 1.81 g (7.98 mmol) of DDQ are added thereto and the mixture is left under stirring at room temperature for 5 min, then evaporated and the residue is purified by flash chromatography eluting with petroleum ether/ethyl acetate 8/2.

Following the same procedure, starting from compound (**Xb**), compound (**Xe**) is prepared.

### Example 6

### 1,4-Dihydro-indeno[1,2-b]pyrrole-3-carbohydrazide (IIa)

A mixture of 2 mmol of ester (**Xa**) and 1.94 ml (40 mmol) of hydrazine is refluxed until completion of the reaction (about 30 min., TLC: CHCl₃/MeOH 9/1), then left to cool and poured into ice. The precipitate is filtered, washed with water and dried in the air.

Following the same procedure, starting from the esters **(Xb, c, f),** hydrazides **(IIb, c, f)** are prepared.

### Example 7

### 3-[[1,3,4]Oxadiazol-2'-yl]-1,4-dihydro-indeno[1,2-b]pyrrole (Ia)

A solution of 2.48 mmol of hydrazide (**IIa**) and 2.73 mmol of ethyl orthoformate in 5 ml of DMF is refluxed for about 8 h (TLC: CHCl₃/MeOH 9/1), then left to cool and poured onto ice. The precipitate is filtered, washed with water and dried in the air.

Following the same procedure, using ethyl orthoacetate or orthobenzoate, compounds **(Ia-g, m**) are prepared, whereas compounds **(Ih, i**, **I)** are prepared from compounds **(Ie, f, g**) following the procedure of Example 4.

**Table 1**

| 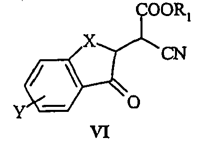 | **Yield** | **M.p.** | **IR** | **¹H-NMR (CDCl₃)** |
|---|---|---|---|---|
| **VIa** | 72.30% | 58-60 °C | 1715 (C=O), | 1.21 (t, 3H, CH₃); 2.58-2.59 (dd, 2H, CH₂); 2.93-3.03 (dd, 1H, CH); 3.40-3.52 (dd, 1H, CH); 4.11 (q, 2H, CH₂); 7.38 (t, 1H, CH); 7.46 (d, 1H, CH); 7.60 (t, 1H, CH); 7.77 (d, 1H, CH) |
| (X = CH₂, Y = H, R₁ = Et) | | | 1745 (C=O), | |
| | | | 2250 (CN) | |
| **VIb** | 96.5% | 54-56 °C | 1680 (C=O), | 1.37 (t, 3H, CH₃); 2.36 (q, 2H, CH₂); 3.12 (t, 2H, CH₂); 4.33 (q, 2H, CH₂); 4.36 (q, 1H, CH); 4.45 (d, 1H, CH); 7.30 (d, 1H, CH); 7.34 (t, 1H, CH); 7.54 (t, 1H, CH); 8.06 (d, 1H, CH) |
| (X= CH₂CH₂, Y = H, R₁ = Et) | | | 1740 (C=O), | |
| | | | 2250 (CN) | |

**Table 2**

| 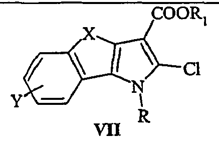 | **Yield** | **M.p.** | **IR** | **¹H-NMR (CDCl₃)** |
|---|---|---|---|---|
| **VIIa** (X = CH₂, Y = H, R₁ = Et, R = H) | 69.8% | 206-208 °C | 1670 (C=O), | 1.40 (t, 3H, CH₃), 3.66 (s, 2H, CH₂), 4.35 (q, 2H, CH₂), 7.11 (t, 1H, CH), 7.25 (t, 1H, CH), 7.42 (d, 1H, CH), 7.47 (d, 1H, CH), 11.5 8 (br s, 1H, NH exchanges with D₂O) |
| | | | 3240 (NH) | |
| **VIIb** | 43.0% | 185-186°C | 1660 (C=O), | 1.38 (t, 3H, CH₃), 2.88-3.05 (m, 4H, CH₂x2), 4.35 (q, 2H, CH₂), 7.10-7.26 (m, 4H, Ph), 8.78 (br s 1H, NH exchanges with D₂O) |
| (X = CH₂CH₂, Y = H, R₁ = Et, R = H) | | | 3210 (NH) | |
| **VIIc** | 95.2% | 102-104 °C | 1700 (C=O) | 1.39 (t, 3H, CH₃), 3.65 (s, 2H, CH₂), 3.87 (s, 3H, CH₃), 4.35 (q, 2H, CH₂), 7.15 (t, 1H, CH), 7.29 (t, 1H, CH), 7.44 (t, 1H, CH), 7.50 (d, 1H, CH) |
| (X = CH₂, Y = H, R₁ = Et, R = CH₃) | | | | |
| **VIId** | 95.3% | 53-55 °C | 1700 (C=O) | 1.38 (t, 3H, CH₃), 2.82-2.98 (m, 4H, CH₂x2), 3.88 (s, 3H, CH₃), 4.34 (q, 2H, CH₂), 7.13-7.41 (m, 4H, CHx4) |
| (X = CH₂CH₂, Y = H, R₁ = Et, R = CH₃) | | | | |

**Table 3**

| 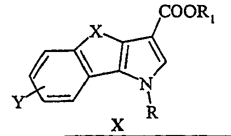 | **Yeld** | **M.p./B.p.** | **IR** | **¹H-NMR** |
|---|---|---|---|---|
| **Xa** | 77.35% | 176-178°C | 1670 (C=O), | (CDCl₃) 1.39 (t, 3H, CH₃); 3.70 (s, 2H, CH₂), 4.35 (q, 2H, CH₂); 7.14 (d, 1H, CH); 7.23 (t, 1H, CH); 7.48 (s, 1H, CH); 7.51 (d, 1H, CH); 8.82 (br s, 1H, NH exchanges with D₂O) |
| (X = CH₂, Y = H, R = H, R₁ = Et) | | | | |
| | | | 3240 (NH) | |
| **Xb** | 71.70% | 140-142°C | 1660 (C=O), | (CDCl₃) 1.36 (t, 3H, CH₃); 2.98 (t, 2H, CH₂); 3.04 (t, 2H, CH₂); 4.32 (q, 2H, CH₂); 7.14-7.25 (m, 4H, CH); 7.43 (s, 1H, CH); 8.65 (br s, 1H, NH exchanges with D₂O) |
| (X = CH2CH2, Y = H, R =H, R1 = Et) | | | | |
| | | | 3250 (NH) | |
| **Xc** | 81.90% | 120-121 °C/0.5 mm Hg | 1695 (C=O) | (CDCl₃) 1.37 (t, 3H, CH₃); 3.66 (s, 2H, CH₂); 3.93 (s, 3H, CH₃); 4.32 (q, 2H, CH₂); 7.16 (d, 1H, CH); 7.27 (s, 1H, CH); 7.30 (t, 1H, CH); 7.44 (d, 1H, CH); 7.50 (d, 1H, CH) |
| (X = CH₂, Y = H, R = CH₃, R₁ = Et) | | | | |
| **Xd** | 70.58% | 59-60 °C/0.5 mm Hg | 1700 (C=O) | (CDCl3) 1.35 (t, 3H, CH3); 2.86 (t, 2H, CH2); 2.98 (t, 2H, CH2); 3.93 (s, 3H, CH3); 4.34 (q, 2H, CH2); 7.10-7.30 (m, 4H, CHx4); 7.43 (d, 1H, CH). |
| (CH₂CH₂, Y = H, R = CH₃, R₁ = Et) | | | | |
| **Xe** | 56.41% | 144.5-145.5 °C | 1660 (C=O), | (CDCl₃+DMSO) 1.39 (t, 3H, CH₃); 4.34 (q, 2H, CH₂); 7.39-7.45 (m, 2H, CHx2); 7.54 (d, 1H, CH); 7.85 (d, 1H, CH, J*_{AB}*= 8.8 Hz); 7.87-7.90 (m, 1H, CH); 8.14 (d, 1H, CH); 8.31 (d, 1H, CH, J*_{AB}* = 8.8 Hz); 12.33 (br s, 1H, NH exchanges with D₂O) |
| (CH=CH, Y = H, R = H, R₁= Et) | | | | |
| | | | 3250 (NH) | |
| **Xf** | 87.88% | 116-118°C | 1690 (C=O) | (CDCl₃) 1.43 (t, 3H, CH₃); 4.24 (s, 3H, CH₃); 4.41 (q, 2H, CH₂); 7.45-7.54 (m, 2H, CHx2); 7.64 (d, 1H, CH, J*_{AB}* = 8.2 Hz); 7.70 (s, 1H, CH); 7.96 (d, 1H, CH); 8.30 (d, 1H, CH, J*_{AB}* = 8.2 Hz); 8.40 (d, 1H, CH) |
| (CH=CH, Y = H, R = CH₃, R₁ = Et) | | | | |

**Table 4**

| 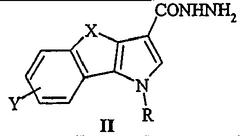 | **Yeld** | **M.p.** | **IR** | ¹**H-NMR (CDCl₃ + DMSO)** |
|---|---|---|---|---|
| **IIa** | 80.0% | 254-256°C | 1650 C=O), | 3.65 (s, 2H, CH₂); 7.06 (t, 1H, CH); 7.22 (t, 1H, CH); 7.39-7.45 (d, 2H, CHx2); 7.88 (s, 1H, CH); 8.68 (br s, 1H, NH exchanges with D₂O) |
| (X=CH₂, Y=H, R=H) | | | 3190-3270 (NH₂) | |
| **IIb** | 80.0% | 268-270°C | 1655 C=O), | 2.66 (t, 2H, CH₂); 2.84 (t, 2H, CH₂); 4.30 (br s, 2H, NH₂ che scambia con D₂O); 6.66 (s, 1H, CH); 7.08 (d, 1H, CH); 7.17 (t, 2H, CHx2); 7.80 (d, 1H, CH); 9.25 (br s, 1H, NH exchanges with D₂O); 11.68 (br s, 1H, NH exchanges with D₂O) |
| (X = CH₂CH₂, Y =H, R = H) | | | 3140-3240 (NH₂), 3300 (NH) | |
| **IIc** | 88.03% | 219-221 °C | 1645 C=O), | 2.85 (br s, 2H, NH₂ exchanges with D₂O); 3.66 (s, 2H, CH₂); 3.93 (s, 3H, CH₃); 7.12 (t, 1H, CH); 7.24-7.34 (m, 2H, CHx2); 7.97 (s, 1H, NH exchanges with D₂O) |
| (X = CH₂, Y = H, R = CH₃) | | | 3300-3320 (NH₂) | |
| **IIf** | 58.1% | 216-218°C | 1625 C=O); | 3.42 (br s, 2H, NH₂ exchanges with D₂O); 4.30 (s, 3H, CH₃); 7.45-7.55 (m, 2H, CHx2); 7.57 (d, 1H, CH, J*_{AB}* = 8.4 Hz); 7.84 (s, 1H, CH); 7.96 (d, 1H, CH); 8.37 (d, 1H, CH, J*_{AB} =* 8.4 Hz); 9.13 (br s, 1H, NH exchanges with D₂O) |
| (CH=CH, Y = H, R = CH₃) | | | 3320 (NH) | |

**Table 5**

| 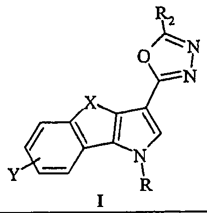 | **Yeld** | **M.p.** | **IR** | **¹H-NMR** |
|---|---|---|---|---|
| **Ia** | 75.0% | 275-278 °C | 1605 (C=N), | (DMSO) 3.68 (s, 2H, CH₂); 7.14 (t, 1H, CH); 7.31 (t, 1H, CH); 7.51 (d, 1H, CH); 7.54 (d, 1H, CH); 7.68 (s, 1H, CH); 9.16 (s, 1H, CH); 12.12 (br s, 1H, NH exchanges with D₂O) |
| (X = CH₂, Y = H, R = H, R₂ = H) | | (EtOH) | | |
| | | | 3180 (NH) | |
| **Ib** | 75.75% | 287-289 °C | 1620 (CN) | (DMSO) 2.55 (s, 3H, CH₃); 3.67 (s, 2H, CH₂), 7.10 (t, 1H, CH); 7.26 (t, 1H, CH); 7.45-7.50 (d, 3H, CHx3); 11.83 (br s, 1H, NH exchanges with D₂O) |
| (X = CH₂, Y = H, R = H, R₂ = CH₃) | | (EtOH) | | |
| **Ic** | 82.14% | 350°C | 1610 (CN) | (CF₃COOD) 3.82 (s, 2H, CH₂); 7.20-7.40 (m, 2H, CHx2); 7.52 (t, 2H, CHx2); 7.68-7.96 (m, 4H, CHx4); 8.16-8.20 (d, 2H, CH₂); 11.60 (br s, 1H, NH exchanges with D₂O) |
| (X = CH₂, Y =H, R =H, R₂ = Ph) | | (dec.) | | |
| **Id** | 78.66% | 175-178°C | 1610 (CN) | (CDCl₃) 3.74 (s, 2H, CH₂); 3.96 (s, 3H, CH₃); 7.20 (t, 1H, CH); 7.32 (s, 1H, CH); 3.74 (s, 1H, CH); 7.47 (d, 1H, CH); 7.53 (d, 1H, CH); 8.83 (s, 1H, CH) |
| (X = CH₂, Y =H, R =CH₃,R₂=H) | | | | |
| **Ie** | 93.33% | 198-200 °C | 1600 (CN), | (CDCl₃ + DMSO) 2.84 (t, 2H, CH₂); 2.91 (t, 2H, CH₂); 2.91 (t, 2H, CH₂); 7.26-7.38 (m, 3H, CHx3); 7.45 (s, 1H, CH); 7.57 (d, 1H, CH); 8.56 (s, 1H, CH); 11.18 (br s, 1H, NH exchanges with D₂0) |
| (X = CH₂CH₂, Y = H, R = H, R₂ = H) | | | 3170 (NH) | |
| **If** | 96.25% | 201-203 °C | 1620 (CN), | (CDCl₃ + DMSO) 2.57 (s, 3H, CH₃); 2.96 (t, 2H, CH₂); 3.05 (t, 2H, CH₂); 7.08-7.24 (m, 3H, CHx3); 7.35-7.45 (m, 2H, CHx2); 10.95 (br s, 1H, NH exchanges with D₂O) |
| (X = CH₂CH₂> Y ₌ H, R = H, R₂ = CH₃) | | | 3250 (NH) | |
| **Ig** | 94 73% | 297-298 °C | 1640 (CN), | (CDCl₃ + DMSO) 3.01 (t, 2H, CH₂); 3.13 (t, 2H, CH₂); 7.09-7.25 (m, 3H, CHx3); 7.49-7.57 (m, 5H, CHx5); 8.06-8.11 (m, 2H, CHx2); 11.56 (br s, 1H, NH exchanges with D₂0) |
| (X = CH₂CH₂, Y = H, R = H, R₂ = Ph) | | | 3170 (NH) | |
| **Ih** | 63.63% | 277-278 °C | 1615 (CN), | (CDCl₃ + DMSO) 7.41-7.55 (m, 3H, CHx3); 7.62 (d, 1H, CH); 7.88 (d, 1H, CH, J*_{AB}* = 6.4 Hz); 8.23 (d, 1H, CH); 8.30 (d, 1H, CH, J*_{AB}* = 6.4 Hz); 8.44 (s, 1H, CH); 12.10 (br s, 1H, NH exchanges with D₂O) |
| (CH=CH, Y = H, R = H, R₂ = H) | | | 3180 (NH) | |
| **Ii** | 90.90% | 326-328 C | 1620(CN) | (CDCl₃ + DMSO) 2.64 (s, 3H, CH₃); 7.44 (s, 1H, CH); 7.45-7.61 (m, 2H, CHx2); 7.67 (d, 1H, CH, *J_{AB}* = 8.8 Hz); 7.93 (d, 1H, CH); 8.32 (d, 1H, CH, J*_{AB}* = 8.8 Hz); 8.38 (d, 1H, CH); 12.03 (br s, 1H, NH exchanges with D₂O) |
| (CH=CH, Y = H, R = H, R₂ = CH₃) | | | | |
| **Il** | 37.50% | 328-329°C | 1630 (CN), | (CDCl₃ + DMSO) 7.48-7.61 (m, 5H, CHx5); 7.62 (s, 1H, CH); 7.68 (d, 1H, CH, *J_{AB} =* 8.8 Hz); 7.96 (d, 1H, CH); 8.11 (d, 1H, CH); 8.15 (d, 1H, CH); 8.26 (d, 1H, CH, J*_{AB}=* 8.8 Hz); 8.44 (d, 1H, CH); 13.42 (br s, 1H, NH exchanges with D₂O) |
| (CH=CH, Y = H, R = H, R₂ =Ph) | | | 3160 (NH) | |
| **Im** | 86.67% | 178-180°C | 1620 (CN) | (CDCl₃) 4.34 (s, 3H, CH₃); 7.48-7.63 (q, 2H, CH×2); 7.70 (d, 1H, CH, J*_{AB}* = 8.4 Hz); 8.02 (d, 1H, CH), 8.38 (d, 1H, CH, J*_{AB}* = 8.4 Hz); 8.40-8.47 (m, 3H, CHx3) |
| (CH=CH, Y = H, R = CH₃, R₂ = H) | | | | |

### Pharmacological section

Biological assays were carried out at the National Cancer Institute according to known experimental protocols [(a) Monks A., Scudiero D., Skehan P., Shoemaker R., Paull K., Vistica D., Hose C., Langley J., Cronise P., Vaigro-Wolff A., Gray-Goodrich M., Campbell H., Mayo J., Boyd M., *J. Natl. Cancer Inst.,* 83, 757-766 (1991). (b) Paull K.D., Shoemaker R.H., Hods L., Monks A., Scudiero D.A., Rubinstein L., Plowman J., Boyd M.R., *J*. *Natl. Cancer Inst.,* 81, 1088-1092 (1989). (c) Boyd M.R., Paull K.D., Rubinstein L.R., Cytotoxic Anticancer Drugs: Models and Concept for Drug discovery, Valeriote F.A., Corbett T., Baker L., Eds., Kluwer Academic Publishers: Amsterdam, 1992, pp. 11].

Table 1 reports the GI₅₀, namely the mean concentration (µM) of substance which causes a 50% inhibition on the cell growth in preliminary tests carried out on three cell lines (MCF7 breast, NCI-H460 microcitoma and SF-268 SNC).

Table 2 shows the GI₅₀ on various tumor cells.
In both cases compounds (**Ic**) and (**Il**) have shown significant antiproliferative activity.

**Table 6**

| | **Ia** | **Ib** | **Ic** | **Ie** | **If** | **Ig** | **Ih** | **Il** | **Im** |
|---|---|---|---|---|---|---|---|---|---|
| R₂ | H | CH₃ | C₆H₆ | H | CH₃ | C₆H₆ | H | C₆H₅ | H |
| X | CH₂ | CH₂ | CH₂ | (CH₂)₂ | (CH₂)₂ | (CH₂)₂ | CH=CH | CH=CH | CH=CH |
| R | H | H | H | H | H | H | H | H | CH₃ |
| *GI₅₀ µM) Mean* | *74.1 1* | *75. 8* | *2.9* | *26. 6* | *N.A.* | *N.A.* | *35. 4* | *1.28* | *33.1* |

| | | | | | | | | | |
|---|---|---|---|---|---|---|---|---|---|
| NA = products inactive in the preliminary tests on three cell lines (MCF7 breast, NCI-H460 microcitoma and SF-268 SNC). | | | | | | | | | |

**Table 7**

| **Cell line** | **Ia** | **Ib** | **Ic** | **Ie** | **If** | **Ig** | **Ih** | **Il** | **Im** |
|---|---|---|---|---|---|---|---|---|---|
| | GI₅₀ | GI₅₀ | GI₅₀ | GI₅₀ | GI₅₀ | GI₅₀ | GI₅₀ | GI₅₀ | GI₅₀ |
| | (µM) | (µM) | (µM) | (µM) | (µM) | (µM) | (µM) | (µM) | (µM) |
| Leukemia | 79.7 | 81.3 | 1.82 | 26.3 | N.A. | N.A. | 44.6 | 0.64 | 54.9 |
| Microcitoma | 57.8 | 93.3 | 3.39 | 21.8 | N.A. | N.A. | 22.3 | 2.36 | 17.7 |
| Colon | 81.2 | 100.0 | 1.00 | 25.7 | N.A. | N.A. | 33.8 | 0.70 | 38.9 |
| SNC | 86.7 | 75.8 | 2.95 | 28.2 | N.A. | N.A. | 32.3 | 1.21 | 23.9 |
| Melanoma | 77.3 | 79.4 | 2.34 | 28.8 | N.A. | N.A. | 33.8 | 2.71 | 43.6 |
| Ovary | 81.2 | 30.2 | 4.57 | 28.2 | N.A. | N.A. | 38.9 | 1.70 | 41.6 |
| Kidney | 67.3 | 91.2 | 6.31 | 27.5 | N.A. | N.A. | 38.0 | 2.04 | 20.8 |
| Prostate | 100 | 100.0 | 4.78 | 32.3 | N.A. | N.A. | 62.3 | 1.28 | 54.9 |
| Breast | 73.2 | 77.6 | 3.16 | 27.5 | N.A. | N.A. | 40.0 | 0.60 | 44.6 |
| *Mean* | *74.1 1* | *75.8* | *2.9* | *26.3* | *N.A.* | *N.A.* | *35.4* | *1.28* | *33.1* |

| | | | | | | | | | |
|---|---|---|---|---|---|---|---|---|---|
| NA = inactive products. | | | | | | | | | |

## Claims

1. Compounds of general formula **(I)** : 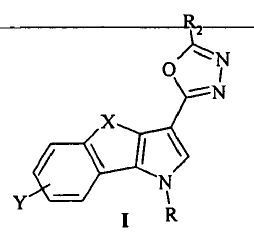 in which:
X is a CH₂, CH₂CH₂ or CH=CH group;
R is hydrogen, straight or branched C₁-C₄ alkyl, or phenyl-C₁-C₂-alkyl;
R₂ is hydrogen, straight or branched C₁-C₄ alkyl, or phenyl, optionally substituted with one more groups, which can be the same or different, selected from halogen (fluorine, chlorine, bromine, iodine), straight or branched C₁-C₄ alkyl, C₁-C₃ alkoxy, trifluoromethyl;
Y is hydrogen, halogen (fluorine, chlorine, bromine, iodine), straight or branched C₁-C₄ alkyl, C₁-C₃ alkoxy, trifluoromethyl.

2. Compounds as claimed in claim 1 wherein C₁-C₄ alkyl is methyl or ethyl; phenyl-C₁-C₂-alkyl is benzyl, optionally substituted on the phenyl ring with one more groups, which can be the same or different, selected from halogen (fluorine, chlorine, bromine, iodine), straight or branched C₁-C₄ alkyl, C₁-C₃ alkoxy, trifluoromethyl; alkoxy-C₁-C₃ is methoxy or ethoxy.

3. Compounds as claimed in claims 1 or 2 in which X is a CH₂ or CH=CH group, R is hydrogen or methyl and R₂ is hydrogen, methyl or optionally substituted phenyl, as defined in claim 1.

4. A compound as claimed in claim 1 selected from:
3-[[1,3,4]oxadiazol-2'-yl]-1,4-dihydro-indeno[1,2-b]pyrrole;
3-[[1,3,4]oxadiazole-5'-methyl-2'-yl]-1,4-dihydro-indeno[1,2-b]pyrrole;
3-[[1,3,4]oxadiazole-5'-phenyl-2'-yl]-1,4-dihydro-indeno[1,2-b]pyrrole;
1-methyl-3-[[1,3,4]oxadiazol-2'-yl]-1,4-dihydro-indeno[1,2-b]pyrrole;
3-[[1,3,4]oxadiazol-2'-yl]-4,5-dihydro-1H-benzo[g]indole;
3-[[1,3,4]oxadiazole-5'-methyl-2'-yl]-4,5-dihydro-1H-Benzo[g]indole;
3-[[1,3,4]oxadiazole-5'-phenyl-2'-yl]-4,5-dihydro-1H-benzo[g]indole;
1-methyl-3-[[1,3,4]oxadiazol-2'-yl]-1H-benzo[g]indole;
3-[[1,3,4]oxadiazol-2'-yl]-1H-benzo[g]indole;
3-[[1,3,4]oxadiazole-5'-methyl-2'-yl]-1H-benzo[g]indole;
3-[[1,3,4]oxadiazole-5'-phenyl-2'-yl]-1H-benzo[g]indole.

5. A compound of formula (**II**) 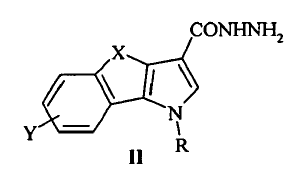 in which X, Y and R are as defined in claim 1, with the exclusion of the compound wherein X is -CH=CH- and Y and R are hydrogen.

6. Compounds of claim 1 as medicaments.

7. The use of the compounds of claim 1 for the preparation of medicaments for the therapy of tumors.

8. The use as claimed in claim 7 in which tumors are: leukemias, colon tumors, breast tumors.

9. Pharmaceutical compositions containing the compounds of claim 1 in admixture with suitable excipients and/or carriers.

## Patentansprüche

1. Verbindungen der allgemeinen Formel (I) 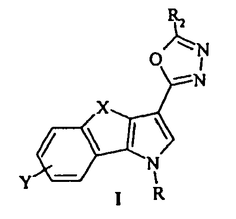 worin:
X eine CH₂-, CH₂CH₂- oder CH=CH-Gruppe ist;
R Wasserstoff, gerad- oder verzweigtkettiges C₁-C₄-Alkyl oder Phenyl-C₁-C₂-alkyl ist;
R₂ Wasserstoff, gerad- oder verzweigtkettiges C₁-C₄-Alkyl oder Phenyl, gegebenenfalls substituiert mit einer oder mehreren Gruppen, die gleich oder verschieden sein können, ausgewählt aus Halogen (Fluor, Chlor, Brom, Iod), gerad- oder verzweigtkettigem C₁-C₄-Alkyl, C₁-C₃-Alkoxy, und Trifluormethyl ist;
Y Wasserstoff, Halogen (Fluor, Chlor, Brom, Iod), gerad- oder verzweigtkettiges C₁-C₄-Alkyl, C₁-C₃-Alkoxy und Trifluormethyl ist.

2. Verbindung nach Anspruch 1, worin C₁-C₄-Alkyl gleich Methyl oder Ethyl ist; Phenyl-C₁-C₂-alkyl gleich Benzyl ist, gegebenenfalls substituiert an dem Phenylring mit einer oder mehreren Gruppen, die gleich oder verschieden sein können, ausgewählt aus Halogen (Fluor, Chlor, Brom, Iod), gerad- oder verzweigtkettigem C₁-C₄-Alkyl, C₁-C₃-Alkoxy, Trifluormethyl; wobei C₁-C₃-alkoxy gleich Methoxy oder Ethoxy ist.

3. Verbindungen nach Anspruch 1 oder 2, worin X eine CH₂- oder CH=CH-Gruppe ist, R Wasserstoff oder Methyl ist und R₂ Wasserstoff, Methyl oder gegebenenfalls substituiertes Phenyl, wie in Anspruch 1 definiert, ist.

4. Verbindung nach Anspruch 1, ausgewählt aus:
3-[[1,3,4]Oxadiazol-2'-yl]-1,4-dihydro-indeno[1,2-b]pyrrol;
3-[[1,3,4]Oxadiazol-5'-methyl-2'-yl]-1,4-dihydro-indeno[1,2-b]pyrrol;
3-[[1,3,4]Oxadiazol-5'-phenyl-2'-yl]-1,4-dihydro-indeno[1,2-b]pyrrol;
1-Methyl-3-[[1,3,4]oxadiazol-2'-yl]-1,4-dihydro-indeno[1,2-b]pyrrol;
3-[[1,3,4]Oxadiazol-2'-yl]-4,5-dihydro-1H-benzo[g]indol;
3-[[1,3,4]Oxadiazol-5'-methyl-2'-yl]-4,5-dihydro-1H-benzo[g]indol;
3-[[1,3,4]Oxadiazol-5'-phenyl-2'-yl]-4,5-dihydro-1H-benzo[g]indol;
1-Methyl-3-[[1,3,4]oxadiazol-2'-yl]-1H-benzo[g]indol;
3-[[1,3,4]Oxadiazol-2'-yl]-1H-benzo[g]indol;
3-[[1,3,4]Oxadiazol-5'-methyl-2'-yl]-1H-benzo[g]indol;
3-[[1,3,4]Oxadiazol-5'-phenyl-2'-yl]-1H-benzo[g]indol.

5. Verbindung der Formel (II) 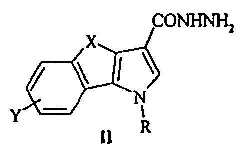 worin X, Y und R wie in Anspruch 1 definiert sind, mit der Ausnahme der Verbindung, worin X gleich -CH=CH- ist und Y und R Wasserstoff sind.

6. Verbindung nach Anspruch 1 als Medikament.

7. Verwendung der Verbindung nach Anspruch 1 zur Herstellung eines Medikaments für die Therapie von Tumoren.

8. Verwendung nach Anspruch 7, wobei die Tumore Leukämie, Colon-Tumore und Brusttumore sind.

9. Pharmazeutische Zusammensetzung, enthaltend die Verbindung nach Anspruch 1 in einem Gemisch mit geeigneten Zusatzstoffen und/oder Trägerstoffen.

## Revendications

1. Composés de formule générale (I): 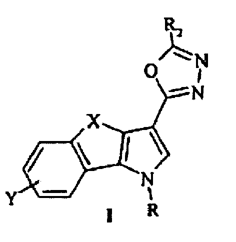 dans laquelle:
X est un groupe CH₂, CH₂CH₂ ou CH=CH ;
R est un atome d'hydrogène, un groupe alkyle en C₁-C₄ linéaire ou ramifié, ou phényl-(alkyle en C₁-C₂) ;
R₂ est un atome d'hydrogène, un groupe alkyle en C₁-C₄ linéaire ou ramifié, ou phényle, éventuellement substitué par un ou plusieurs groupe(s), qui peut(peuvent) être identique(s) ou différent(s), choisi(s) parmi un atome d'halogène (fluor, chlore, brome, iode), un groupe alkyle en C₁-C₄ linéaire ou ramifié, un groupe alcoxy en C₁-C₃, un groupe trifluorométhyle ;
Y est un atome d'hydrogène, un atome d'halogène (fluor, chlore, brome, iode), un groupe alkyle en C₁-C₄ linéaire ou ramifié, un groupe alcoxy en C₁-C₃, un groupe trifluorométhyle.

2. Composés selon la revendication 1, dans lesquels le groupe alkyle en Cₗ-C₄ est le groupe méthyle ou éthyle ; le groupe phényl-(alkyle en C₁-C₂) est le groupe benzyle, éventuellement substitué sur le cycle phényle par un ou plusieurs groupe(s), qui peut(peuvent) être identique(s) ou différent(s), choisi(s) parmi un atome d'halogène (fluor, chlore, brome, iode), un groupe alkyle en C₁-C₄ linéaire ou ramifié, un groupe alcoxy en C₁-C₃, trifluorométhyle ; le groupe alcoxy en C₁-C₃ est le groupe méthoxy ou éthoxy.

3. Composés selon les revendications 1 ou 2, dans lesquels X est un groupe CH₂ ou CH=CH, R est un atome d'hydrogène ou un groupe méthyle et R₂ est un atome d'hydrogène, un groupe méthyle ou un groupe phényle éventuellement substitué, tel que défini selon la revendication 1.

4. Composé selon la revendication 1, choisi parmi :
le 3-[[1,3,4]oxadiazol-2'-yl]-1,4-dihydro-indéno[1,2-b]pyrrole ;
le 3-[[1,3,4]oxadiazole-5'-méthyl-2'-yl]-1,4-dihydro-indéno[1,2-b]pyrrole ;
le 3-[[1,3,4]oxadiazole-5'-phényl-2'-yl]-1,4-dihydro-indéno[1,2-b]pyrrole ;
le 1-méthyl-3-[[-[[1,3,4]oxadiazol-2'-yl]-1,4-dihydro-indéno[1,2-b]pyrrole ;
le 3-[[1,3,4]oxadiazol-2'-yl]-4,5-dihydro-1H-benzo[g]indole ;
le 3-[[1,3,4]oxadiazole-5'-méthyl-2'-yl]-4,5-dihydro-1H-benzo[g]indole ;
le 3-[[1,3,4]oxadiazole-5'-phényl-2'-yl]-4,5-dihydro-1H-benzo[g]indole ;
le 1-méthyl-3-[[1,3,4]oxadiazol-2'-yl]-1H-benzo[g]indole ;
le 3-[[1,3,4]oxadiazol-2'-yl]-1H-benzo[g]indole;
le 3-[[1,3,4]oxadiazole-5'-méthyl-2'-yl]-1H-benzo[g]indole;
le 3-[[1,3,4]oxadiazole-5'-phényl-2'-yl]-1H-benzo[g]indole.

5. Composé de formule (II) 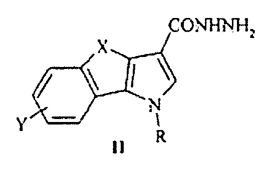 dans laquelle X, Y et R sont tels que définis selon la revendication 1, à l'exclusion du composé dans lequel X est -CH=CH- et Y et R sont l'atome d'hydrogène.

6. Composés selon la revendication 1 comme médicaments.

7. Utilisation des composés selon la revendication 1 pour la préparation de médicaments pour la thérapie des tumeurs.

8. Utilisation selon la revendication 7, dans laquelle les tumeurs sont : les leucémies, les tumeurs du colon, les tumeurs du sein.

9. Compositions pharmaceutiques contenant les composés selon la revendication 1, en mélange avec des excipients et/ou des supports appropriés.
